# FASCICULE DE BREVET EUROPEEN

(11) **EP 0 625 147 B1**
(45) Date de publication et mention de la délivrance du brevet: **28.08.1996**
(21) Numéro de dépôt: 93904150.5
(22) Date de dépôt: 04.02.1993
(51) Int. Cl.: C07D 305/14

(54) **PROCEDE DE PREPARATION DE DERIVES DU TAXANE**
VERFAHREN ZUR HERSTELLUNG VON TAXAN-DERIVATEN
METHOD FOR PREPARING TAXANE DERIVATIVES

(30) Priorité: 07.02.1992 FR 9201379
(43) Date de publication de la demande: 23.11.1994
(73) Titulaire: RHONE-POULENC RORER S.A., 92160 Antony (FR)
(72) Inventeur: FOUQUE, Elie, F-94200 Ivry-sur-Seine (FR); MAS, Jean-Manuel, F-69100 Villeurbanne (FR)
(74) Mandataire: Pilard, Jacques
(86) Numéro de dépôt international: FR9300110
(87) Numéro de publication internationale: WO9316059

(56) Documents cités:
- EP-A- 0 336 840
- EP-A- 0 336 841

## Description

La présente invention concerne un nouveau procédé de préparation des dérivés du taxane de formule générale : 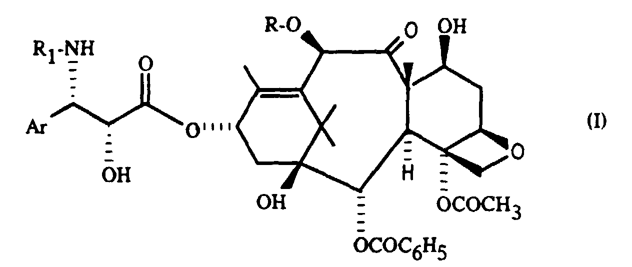 dans laquelle Ar représente un radical aryle, R représente un atome d'hydrogène ou le radical acétyle et R₁ représente un radical benzoyle ou tert.butoxycarbonyle, qui présentent des propriétés antitumorales remarquables.

D'après les brevets américains US 4 924 011 et US 4 924 012, il est connu de préparer les dérivés du taxane de formule générale (I) par estérification d'un dérivé de la baccatine III ou de la désacétyl-10 baccatine III de formule générale : 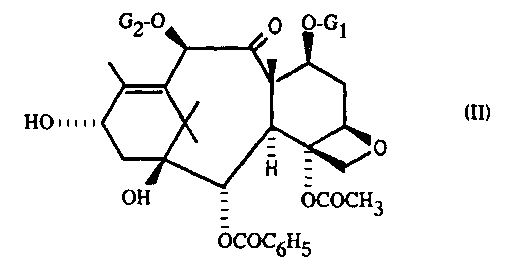 dans laquelle G₁ représente un groupement protecteur de la fonction hydroxy tel que le radical trichloro-2,2,2 éthoxycarbonyle ou un radical trialcoylsilyle dont chaque partie alcoyle contient 1 à 4 atomes de carbone et G₂ représente le radical acétyle ou un groupement protecteur de la fonction hydroxy tel que le radical trichloro-2,2,2 éthoxycarbonyle, au moyen d'un acide de formule générale : 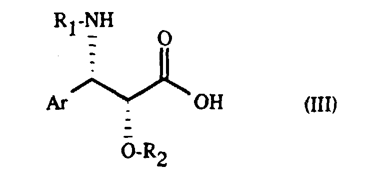 dans laquelle Ar et R₁ sont définis comme précédemment et R₂ représente un groupement protecteur de la fonction hydroxy tel qu'un radical méthoxyméthyle, éthoxy-1 éthyle, benzyloxyméthyle, (β-triméthylsilyléthoxy) méthyle, tétrahydropyrannyle, trichloro-2,2,2 éthoxyméthyle ou trichloro-2,2,2 éthoxycarbonyle, suivie du remplacement des groupements protecteurs G₁, G₂ et R₂ du produit obtenu par des atomes d'hydrogène.

L'estérification est effectuée en présence d'un agent de condensation tel qu'un carbodiimide comme le dicyclohexylcarbodiimide ou un carbonate réactif comme le dipyridyl-2 carbonate et d'un agent d'activation tel qu'une dialcoylaminopyridine comme la diméthylamino-4 pyridine en opérant dans un solvant organique aromatique tel que le benzène, le toluène, les xylènes, l'éthylbenzène, l'isopropylbenzène ou le chlorobenzène à une température comprise entre 60 et 90°C.

Le remplacement des groupements protecteurs par des atomes d'hydrogène s'effectue au moyen de zinc dans l'acide acétique ou par hydrolyse en milieu acide.

Il a maintenant été trouvé, et c'est ce qui fait l'objet de la présente invention, que l'estérification de l'alcool de formule générale (II) au moyen de l'acide de formule générale (III) peut être réalisée à une température comprise entre -10 et 60°C (60°C non inclus), de préférence comprise entre 20 et 35°C en opérant dans un solvant organique choisi parmi les éthers tels que le tétrahydrofuranne, l'éther diisopropylique, le méthyl tert.butyléther ou le dioxanne, les cétones telles que la méthylisobutylcétone, les nitriles tels que l'acétonitrile, les esters tels que l'acétate d'éthyle, l'acétate d'isopropyle ou l'acétate de n.butyle, les hydrocarbures aliphatiques tels que le pentane, l'hexane ou l'heptane, les hydrocarbures aliphatiques chlorés tels que le dichlorométhane ou le dichloro-1,2 éthane et les hydrocarbures aromatiques tels que le benzène, le toluène ou les xylènes. D'un intérêt tout particulier sont les esters et les hydrocarbures aromatiques.

Généralement, l'estérification est effectuée en présence d'un agent de condensation tel qu'un carbodiimide comme le dicyclohexylcarbodiimide et d'un agent d'activation tel qu'une aminopyridine comme la diméthylamino-4 pyridine ou la pyrrolidino-4 pyridine.

Il est avantageux de réaliser l'estérification en utilisant un excès d'acide de formule générale (III) par rapport à l'alcool de formule générale (II) mais cependant la réaction peut aussi être conduite en utilisant une quantité stoechiométrique d'acide de formule générale (III) et d'alcool de formule générale (II). L'agent de condensation est utilisé généralement en quantité stoechiométrique par rapport à l'acide de formule générale (III) et l'agent d'activation représente une quantité stoechiométrique ou inférieure par rapport à l'alcool de formule générale (II).

Le procédé selon l'invention, du fait qu'il est mis en oeuvre à une température inférieure à celle des procédés antérieurement connus, permet d'obtenir des rendements plus élevés en ester du fait de la meilleure stabilité de l'acide de formule générale (III) dans le mélange réactionnel et de la régression des réactions secondaires.

Les exemples suivants illustrent la présente invention.

### EXEMPLE 1

Dans un erlenmeyer de 20 cm3, on introduit 1,0045 g d'acétoxy-4 benzoyloxy-2α époxy-5β,20 dihydroxy-1,13α oxo-9 bis-(trichloro-2,2,2 éthoxy) carbonyloxy-7β,10β taxène-11 à 96 % (soit 1,08 mmole), 1,1545 g d'acide (éthoxy-1 éthoxy)-2 tert.butoxycarbonylamino-3 phényl-3 propionique-(2R,3S) à 100 % (soit 3,3 mmoles), 0,6669 g de dicyclohexylcarbodiimide à 99 % (soit 3,2 mmoles), 0,0742 g de pyrrolidino-4 pyridine à 98 % (soit 0,49 mmole) et 6 cm3 de toluène anhydre. On agite vigoureusement pendant 72 heures en maintenant la température à -10°C.

Le dosage du milieu réactionnel par chromatographie liquide haute performance montre que le milieu contient 1,1370 g de tert.butoxycarbonylamino-3 phényl-3 (éthoxy-1 éthoxy)-2" propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 0,91 mmole) et 0,1705 g de tert.butoxycarbonylamino-3 phényl-3 (éthoxy-1 éthoxy)-2" propionate-(2S,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 0,14 mmole).

Le rendement total est de 97 % avec un taux d'épimérisation de 12,7 %.

### EXEMPLE 2

Dans un réacteur double-enveloppe en verre de 500 cm3 équipé d'une arrivée d'azote, d'une sonde de température et d'un réfrigérant, on introduit 50,011 g d'acétoxy-4 benzoyloxy-2α époxy-5β,20 dihydroxy-1,13α oxo-9 bis-(trichloro-2,2,2 éthoxy) carbonyloxy-7β,10β taxène-11 à 96 % (soit 53,6 mmoles), 56,81 g d'acide (éthoxy-1 éthoxy)-2 tert.butoxycarbonylamino-3 phényl-3 propionique-(2R,3S) à 100 % (soit 160,7 mmoles), 33,54 g de dicyclohexylcarbodiimide à 99 % (soit 160,9 mmoles), 1,79 g de pyrrolidino-4 pyridine à 98 % (soit 11,8 mmoles) et 299 cm3 de toluène anhydre. On agite vigoureusement pendant 12 heures en maintenant à une température voisine de 25°C.

Le dosage du milieu réactionnel par chromatographie liquide haute performance montre que le milieu contient 57,00 g de tert.butoxycarbonylamino-3 phényl-3 (éthoxy-1 éthoxy)-2" propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 45,7 mmoles) et 8,52 g de tert.butoxycarbonylamino-3 phényl-3 (éthoxy-1 éthoxy)-2" propionate-(2S,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 6,8 mmoles).

Le rendement total est de 98 % avec un taux d'épimérisation de 13,0 %.

### EXEMPLES 3 à 19

Dans un erlenmeyer de 10 cm3, on introduit 250 mg d'acétoxy-4 benzoyloxy-2α époxy-5β,20 dihydroxy-1,13α oxo-9 bis-(trichloro-2,2,2 éthoxy) carbonyloxy-7β,10β taxène-11 à 96 % (soit 0,27 mmole), 284 mg d'acide (éthoxy-1 éthoxy)-2 tert.butoxycarbonylamino-3 phényl-3 propionique-(2R,3S) à 100 % (soit 0,80 mmole), 190 mg de dicyclohexylcarbodiimide à 99 % (soit 0,91 mmole), 9 mg de diméthylamino-4 pyridine à 98 % (soit 0,07 mmole) et 3 cm3 de solvant. On agite vigoureusement en maintenant le milieu réactionnel à une température voisine de 30°C.

Après de 16 à 24 heures d'agitation, le dosage du milieu réactionnel par chromatographie liquide haute performance permet de calculer le rendement d'estérification et le taux d'épimérisation.

Les résultats qui sont obtenus avec différents solvants sont rassemblés dans le tableau suivant.

| Exemple | Solvant | Durée de la réaction (heures) | Rendement d'estérification | Taux d'épimérisation (%) |
|---|---|---|---|---|
| 3 | Tétrahydrofuranne | 17,0 | 24,5 | 7,3 |
| 4 | Diisopropyléther | 23,5 | 87,4 | 14,2 |
| 5 | Méthyltert.butyléther | 17,0 | 87,3 | 12,5 |
| 6 | Dioxanne | 16,2 | 19,6 | 10,8 |
| 7 | Acétonitrile | 23,5 | 58,8 | 46,9 |
| 8 | Benzène | 16,2 | 96,0 | 12,6 |
| 9 | Toluène | 23,5 | 98,0 | 13,9 |
| 10 | Métaxylène | 17,0 | 97,8 | 14,4 |
| 11 | Anisole | 16,2 | 94,1 | 19,2 |
| 12 | Chlorobenzène | 16,2 | 96,0 | 17,5 |
| 13 | Cyclohexane | 17,0 | 61,7 | 15,8 |
| 14 | Pentane | 16,2 | 51,0 | 31,2 |
| 15 | n-Hexane | 23,5 | 42,1 | 29,7 |
| 16 | Heptane | 16,2 | 37,2 | 21,5 |
| 17 | Dichloro-1,2 éthane | 17,0 | 92,8 | 28,9 |
| 18 | Dichlorométhane | 17,0 | 83,7 | 26,6 |
| 19 | Méthylisobutylcétone | 16,2 | 58,8 | 17,4 |
| 20 | Acétate d'éthyle | 8 | 75 | 12,7 |

### EXEMPLE 21

Dans un erlenmeyer de 10 cm3, on charge 503,6 mg d'acétoxy-4 benzoyloxy-2α époxy-5β,20 dihydroxy-1,13α oxo-9 bis-(trichloro-2,2,2 éthoxy) carbonyl-7β,10β taxène-11 à 96 % (soit 0,54 mmole), 579,0 mg d'acide (éthoxy-1 éthoxy)-2 tert.butoxycarbonylamino-3 phényl-3 propionique-(2R,3S) à 99 % (soit 1,62 mmole), 357,8 mg de dicyclohexylcarbodiimide à 99 % (soit 1,72 mmole), 45,5 mg de pyrrolidino-4 pyridine à 98 % (soit 0,30 mmole) et 3 cm3 de toluène anhydre. On agite vigoureusement pendant 5 heures 20 minutes en maintenant la température à 45°C.

Le dosage du milieu réactionnel par chromatographie liquide haute performance montre que le milieu contient 559,5 mg de tert.butoxycarbonylamino-3 phényl-3 (éthoxy-1 éthoxy)-2" propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 0,45 mmole) et 90,8 mg de tert.butoxycarbonylamino-3 phényl-3 (éthoxy-1 éthoxy)-2" propionate-(2S,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α (soit 0,07 mmole).

Le rendement total est de 97 % avec un taux d'épimérisation de 13,9 %.

## Revendications

1. Procédé de préparation de dérivés du taxane de formule générale : 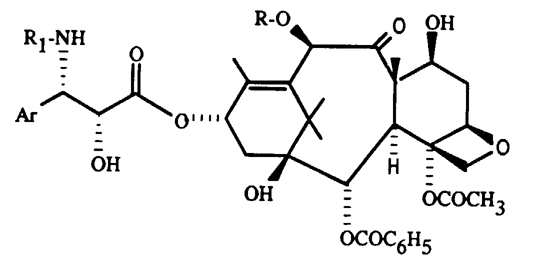 dans laquelle Ar représente un radical aryle, R représente un atome d'hydrogène ou le radical acétyle et R₁ représente un radical benzoyle ou tert.butoxycarbonyle par estérification d'un dérivé de la baccatine III ou de la désacétyl-10 baccatine III de formule générale : 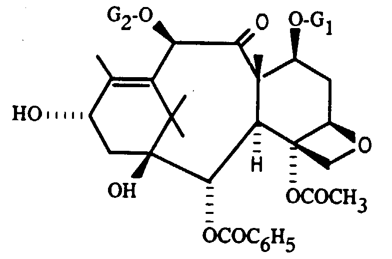 dans laquelle G₁ représente un groupement protecteur de la fonction hydroxy tel qu'un radical trichloro-2,2,2 éthoxycarbonyle ou un radical trialkylsilyle dont chaque partie alkyle contient 1 à 4 atomes de carbone et G₂ représente le radical acétyle ou un groupement protecteur de la fonction hydroxy tel que le radical trichloro-2,2,2 éthoxycarbonyle, au moyen d'un acide de formule générale : 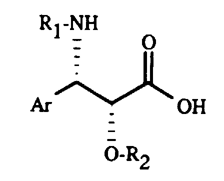 dans laquelle Ar et R₁ sont définis comme précédemment et R₂ représente un groupement protecteur de la fonction hydroxy tel qu'un radical méthoxyméthyle, éthoxy-1 éthyle, benzyloxyméthyle, (β-triméthylsilyléthoxy) méthyle, tétrahydropyrranyle, trichloro-2,2,2 éthoxyméthyle ou trichloro-2,2,2 éthoxycarbonyle, suivie du remplacement des groupements protecteurs G₁, G₂ et G₂ du produit obtenu par des atomes d'hydrogène, caractérisé en ce que l'estérification est effectuée à une température comprise entre -10 et 60°C (60°C non inclus).

2. Procédé selon la revendication 1 caractérisé en ce que l'estérification est effectuée dans un solvant organique choisi parmi les éthers, les cétones, les esters, les nitriles, les hydrocarbures aliphatiques, les hydrocarbures aliphatiques chlorés, les hydrocarbures aromatiques.

3. Procédé selon la revendication 2 caractérisé en ce que le solvant est choisi parmi les esters, les hydrocarbures aromatiques.

4. Procédé selon l'une des revendications 1, 2 ou 3 caractérisé en ce que l'on opère en présence d'un agent de condensation et d'un agent d'activation.

5. Procédé selon la revendication 4 caractérisé en ce que l'agent de condensation est un carbodiimide.

6. Procédé selon la revendication 4 caractérisé en ce que l'agent d'activation est une aminopyridine.

7. Procédé selon la revendication 5 caractérisé en ce que le carbodiimide est le dicyclohexylcarbodiimide.

8. Procédé selon la revendication 6 caractérisé en ce que l'aminopyridine est la diméthylamino-4 pyridine ou la pyrrolidino-4 pyridine.

## Claims

1. Process for the preparation of taxane derivatives of general formula: 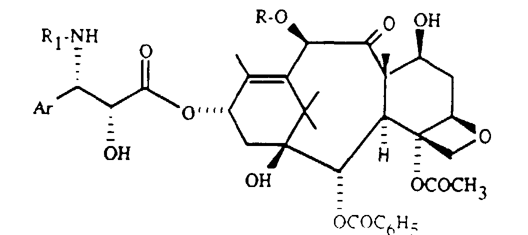 in which Ar represents an aryl radical, R represents a hydrogen atom or an acetyl radical and R₁ represents a benzoyl or tert-butoxycarbonyl radical, by esterification of a derivative of baccatin III or of 10-deacetylbaccatin III of general formula: 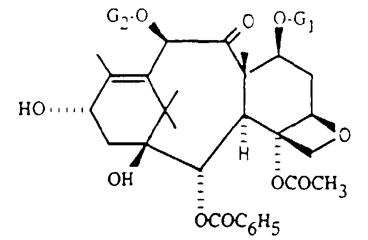 in which G₁ represents a protecting group of the hydroxyl functional group, such as a 2,2,2-trichloroethoxycarbonyl radical or a trialkylsilyl radical, each alkyl part of which contains 1 to 4 carbon atoms, and G₂ represents an acetyl radical or a protecting group of the hydroxyl functional group, such as a 2,2,2-trichloroethoxycarbonyl radical, using an acid of general formula: 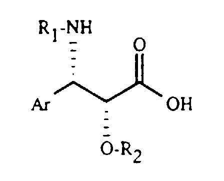 in which Ar and R₁ are defined as above and R₂ represents a protecting group of the hydroxyl functional group, such as a methoxymethyl, 1-ethoxyethyl, benzyloxymethyl, (β-trimethylsilylethoxy)methyl, tetrahydropyranyl, 2,2,2-trichloroethoxymethyl or 2,2,2-trichloroethoxycarbonyl radical, followed by replacement of the protecting groups G₁, G₂ and R₂ of the product obtained by hydrogen atoms, characterised in that esterification is carried out at a temperature of between -10 and 60°C (60°C not included).

2. Process according to claim 1, characterised in that esterification is carried out in an organic solvent chosen from the ethers, ketones, esters, nitriles, aliphatic hydrocarbons, chlorinated aliphatic hydrocarbons and aromatic hydrocarbons.

3. Process according to claim 2, characterised in that the solvent is chosen from the esters and aromatic hydrocarbons.

4. Process according to one of claims 1, 2 and 3, characterised in that the esterification is carried out in the presence of a condensation agent and of an activating agent.

5. Process according to claim 4, characterised in that the condensation agent is a carbodiimide.

6. Process according to claim 4, characterised in that the activating agent is an aminopyridine.

7. Process according to claim 5, characterised in that the carbodiimide is dicyclohexylcarbodiimide.

8. Process according to claim 6, characterised in that the aminopyridine is 4-dimethylaminopyridine or 4-pyrrolidinopyridine.

## Patentansprüche

1. Verfahren zur Herstellung von Taxan-Derivaten der allgemeinen Formel 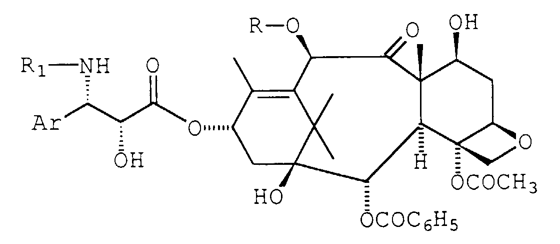 in der Ar einen Arylrest darstellt, R ein Wasserstoffatom oder den Acetylrest bedeutet und R₁ ein Benzoylrest oder ein tert.-Butoxycarbonylrest ist, durch Veresterung eines Derivates von Baccatin III oder von 10-Desacetyl-Baccatin III der allgemeinen Formel 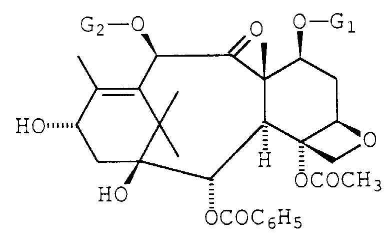 in der G₁ eine Schutzgruppe für die Hydroxyfunktion darstellt, wie ein 2,2,2-Trichlor-ethoxycarbonylrest oder ein Trialkylsilylrest, dessen Alkylteil 1 bis 4 Kohlenstoffatome enthält, und G₂ einen Acetylrest oder eine Schutzgruppe für die Hydroxyfunktion bedeutet, wie der 2,2,2-Trichlor-ethoxycarbonylrest, mit Hilfe einer Säure der allgemeinen Formel 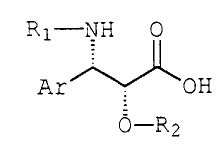 in der Ar und R₁ wie oben definiert sind und R₂ eine Schutzgruppe für die Hydroxyfunktion darstellt, wie einer der Reste Methoxymethyl, 1-Ethoxyethyl, Benzyloxymethyl, (β-Trimethylsilylethoxy)-methyl, Tetrahydropyranyl, 2,2,2-Trichlor-ethoxymethyl oder 2,2,2-Trichlor-ethoxycarbonyl, gefolgt von dem Austausch der Schutzgruppen G₁, G₂ und R₂ des erhaltenen Produktes durch Wasserstoffatome, dadurch gekennzeichnet, daß die Veresterung bei einer Temperatur zwischen -10 °C und 60 °C (60 °C nicht eingeschlossen) durchgeführt wird.

2. Verfahren nach Anspruch 1, dadurch gekennzeichnet, daß die Veresterung in einem organischen Lösungsmittel durchgeführt wird, ausgewählt unter den Ethern, den Ketonen, den Estern, den Nitrilen, den aliphatischen Kohlenwasserstoffen, den chlorierten aliphatischen Kohlenwasserstoffen und den aromatischen Kohlenwasserstoffen.

3. Verfahren nach Anspruch 2, dadurch gekennzeichnet, daß das Lösungsmittel unter den Estern und den aromatischen Kohlenwasserstoffen ausgewählt wird.

4. Verfahren nach einem der Ansprüche 1, 2 oder 3, dadurch gekennzeichnet, daß man in Anwesenheit eines Kondensationsmittels und eines Aktivierungsmittels arbeitet.

5. Verfahren nach Anspruch 4, dadurch gekennzeichnet, daß das Kondensationsmittel ein Carbodiimid ist.

6. Verfahren nach Anspruch 4, dadurch gekennzeichnet, daß das Aktivierungsmittel ein Aminopyridin ist.

7. Verfahren nach Anspruch 5, dadurch gekennzeichnet, daß das Carbodiimid das Dicyclohexylcarbodiimid ist.

8. Verfahren nach Anspruch 6, dadurch gekennzeichnet, daß das Aminopyridin das 4-Dimethylaminopyridin oder das 4-Pyrrolidinopyridin ist.
